# EUROPEAN PATENT APPLICATION

(11) **EP 3 076 169 A1**
(43) Date of publication of application: **05.10.2016**
(21) Application number: 13897388.8
(22) Date of filing: 12.11.2013
(51) Int. Cl.: G01N 29/44, G01M 99/00, G01H 13/00, G01V 1/00, G01W 1/02

(54) **ANALYSIS DEVICE, ANALYSIS METHOD, AND ANALYSIS PROGRAM**

(71) Applicant: NEC Corporation, Tokyo 108-8001 (JP)
(72) Inventor: OCHIAI, Katsuhiro, Tokyo 108-8001 (JP)
(74) Representative: Betten & Resch
(86) International application number: PCT/JP2013/006639
(87) International publication number: WO 2015/071925

(57) **Abstract**

An analysis device 101 includes: a first sensor 102 for acquiring waveform data generated from an object; a second sensor 103 for acquiring time-series data of an element that affects a change in state of the object; frequency transform means 105 for acquiring a frequency of the waveform data; feature value calculation means 107 for calculating a feature value based on the frequency; time-series arrangement means 108 for assigning a time corresponding to the time-series data, to the feature value; and correlation check means 113 for checking a correlation between the feature value and the time-series data.

## Description

### Technical Field

The present invention relates to an analysis device, analysis method, and analysis program for time-series data using frequency feature values.

### Background Art

To detect the occurrence of sediment disasters, a method of estimating soil moisture content from rainfall, called the tank model, has been commonly used (for example, see Non Patent Literature (NPL) 1). With this method, in the case where the estimate is not less than a predetermined value, it is determined that there is a possibility of occurrence of a sediment disaster, and evacuation warnings and the like are issued.

As another method of detecting the occurrence of sediment disasters, a technique of semi-infinite length slope stability analysis has been used (for example, see NPL 2). The technique of semi-infinite length slope stability analysis is a method of modeling slope stability from saturated moisture content in soil and estimating the degree of risk from rainfall.

Patent Literature (PTL) 1 discloses a sediment disaster prediction system that generates vibration at one location of soil, measures vibration at a predetermined distance away from the location, analyzes the measurement result, and outputs volume water content and the like.

### Citation List

### Patent Literature

PTL 1: Japanese Patent Application Laid-Open No. 2005-30843 (paragraphs 0013 to 0015)

### Non Patent Literature

NPL 1: Ishihara, Y. and S. Kobatake (1979): "Runoff Model for Flood Forecasting", Bull. D. P. R. I., Kyoto Univ., 29, pp. 27-43
NPL 2: H. Yamashita, S. Nishiyama, and S. Tanaka (2005): "Stability Computation for Calculating Current Safety Factor of Steep Slope", JGCA "Technical e-Forum 2005", Sendai

### Summary of Invention

### Technical Problem

The estimation technique by the tank model and the semi-infinite length slope stability analysis technique have the following problem: To perform prediction on a land-by-land basis, soil properties, topographic features, etc. need to be taken into consideration, and soil components need to be analyzed. The sediment disaster prediction system described in PTL 1 needs geological data in order to calculate volume water content. Thus, the above-mentioned techniques are problematic in that investigating soil components and the like requires time and cost.

In view of this, the present invention has an object of providing an analysis device, analysis method, and analysis program capable of detecting a change in the state of an object easily and accurately.

### Solution to Problem

An analysis device according to the present invention includes: a first sensor for acquiring waveform data generated from an object; a second sensor for acquiring time-series data of an element that affects a change in state of the object; frequency transform means for acquiring a frequency of the waveform data; feature value calculation means for calculating a feature value based on the frequency; time-series arrangement means for assigning a time corresponding to the time-series data, to the feature value; and correlation check means for checking a correlation between the feature value and the time-series data.

An analysis method according to the present invention includes: acquiring waveform data generated from an object; acquiring time-series data of an element that affects a change in state of the object; acquiring a frequency of the waveform data; calculating a feature value based on the frequency; assigning a time corresponding to the time-series data, to the feature value; and checking a correlation between the feature value and the time-series data.

An analysis program according to the present invention causes a computer to execute: a frequency transform process of acquiring a frequency of waveform data generated from an object; a feature value calculation process of calculating a feature value based on the frequency; a time-series arrangement process of assigning a time corresponding to time-series data of an element that affects a change in state of the object, to the feature value; and a correlation check process of checking a correlation between the feature value and the time-series data.

### Advantageous Effects of Invention

According to the present invention, it is possible to detect a change in the state of an object easily and accurately.

### Brief Description of Drawings

[Fig. 1] Fig. 1 is a block diagram depicting the structure of a first exemplary embodiment of an analysis device according to the present invention.
[Fig. 2] Fig. 2 is a flowchart depicting the operation of the first exemplary embodiment of the analysis device according to the present invention.
[Fig. 3] Fig. 3 is a block diagram depicting the structure of an analysis device in Example 1.
[Fig. 4] Fig. 4 is a flowchart depicting the operation of the analysis device in Example 1.
[Fig. 5] Fig. 5 is a block diagram depicting the structure of a second exemplary embodiment of the analysis device according to the present invention.
[Fig. 6] Fig. 6 is a flowchart depicting the operation of the second exemplary embodiment of the analysis device according to the present invention.
[Fig. 7] Fig. 7 is a block diagram depicting the structure of an analysis device in Example 2.
[Fig. 8] Fig. 8 is a flowchart depicting the operation of the analysis device in Example 2.
[Fig. 9] Fig. 9 is a block diagram depicting the structure of main parts of the analysis device according to the present invention.

### Description of Embodiment

The following describes an analysis device according to exemplary embodiments of the present invention, with reference to drawings.

### Exemplary Embodiment 1

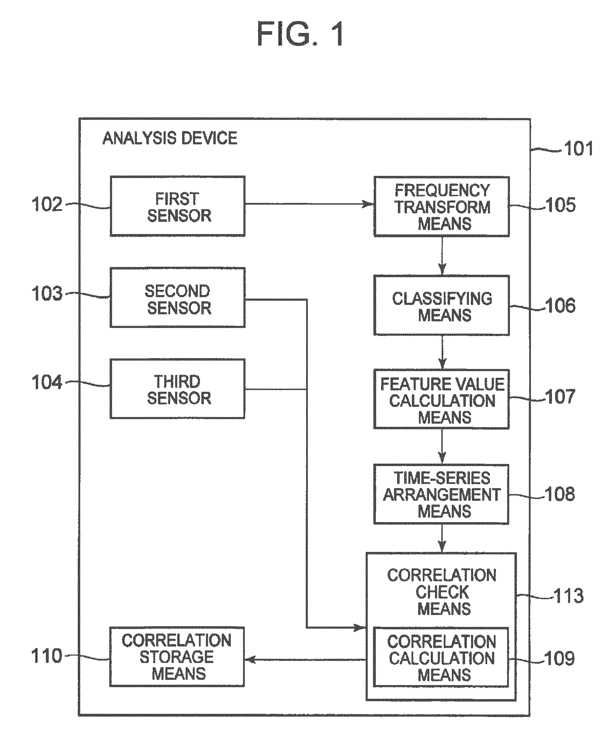

Fig. 1 is a block diagram depicting the structure of an analysis device in a first exemplary embodiment (Exemplary Embodiment 1). An analysis device 101 in this exemplary embodiment is an analysis device in a modeling stage.

The analysis device 101 includes a first sensor 102, a second sensor 103, and a third sensor 104. The analysis device 101 also includes frequency transform means 105, classifying means 106, feature value calculation means 107, time-series arrangement means 108, correlation check means 113, and correlation storage means 110. The correlation check means 113 includes correlation calculation means 109.

The frequency transform means 105, the classifying means 106, the feature value calculation means 107, the time-series arrangement means 108, and the correlation check means 113 are realized, for example, by an information processing device such as a central processing unit (CPU) operating according to a program or hardware designed to perform specific computation and the like. The program is computer-readable, and is stored in a non-transitory information storage medium.

The first sensor 102 is connected to the frequency transform means 105. The first sensor 102 acquires waveform data of light, sound, vibration, or the like generated from an object. The waveform data is time-series data. As an example, in the case where the first sensor 102 is a vibration sensor, the waveform data is waveform data of the vibration of the object. As another example, the waveform data may be reflected light or transmitted light in the case where the object is radiated with light. As another example, the waveform data may be waveform data of sound generated from the object, which is obtained by an ultrasound method, an impact elastic wave method, a hammering method, or the like.

The sensor that outputs data to the frequency transform means 105 is not limited only to the first sensor 102, and may be a plurality of sensors. In such a case, the frequency transform means 105, the classifying means 106, the feature value calculation means 107, and the time-series arrangement means 108 perform the respective processes for sensor data of each sensor, and the correlation calculation means 109 combines these multiple data and calculates mutual correlation.

The second sensor 103 and the third sensor 104 are connected to the correlation calculation means 109. The second sensor 103 and the third sensor 104 each acquire time-series data of an element that affects a change in the state of the object. For example, the second sensor 103 and the third sensor 104 acquire time-series data of rainfall, temperature, humidity, sunlight intensity, object inclination, pressure on the object, soil moisture content, etc. Although two sensors (the second sensor 103 and the third sensor 104) acquire time-series data in this exemplary embodiment, the number of sensors that acquire time-series data may be one, or three or more.

The frequency transform means 105 acquires the frequency of the waveform data, and outputs the frequency data to the classifying means 106.

The classifying means 106 classifies the frequency-transformed data by a predetermined frequency width, and outputs the data to the feature value calculation means 107. The frequency width may be any frequency width. The analysis device 101 does not necessarily need to include the classifying means 106. For example, in the case where the feature value calculation means 107 calculates a natural frequency as a feature value, the analysis device 101 does not need to perform classifying. The classifying means 106 may exclude unnecessary frequency bands from the classifying process. In this case, the analysis device 101 may omit subsequent processes such as feature value calculation.

The feature value calculation means 107 calculates, for each classifying range, a feature value based on the classified frequency, and outputs the calculated feature value to the time-series arrangement means 108. The feature value calculation means 107 calculates the feature value of each set of data, by calculating the average value, maximum value, dispersion, deviation, root mean square (RMS), etc. of each classifying range of the frequency. The feature value calculation means 107 may determine a natural frequency (also referred to as a dominant frequency) in the frequency data, and set the frequency value of the natural frequency as the frequency feature value.

The time-series arrangement means 108 acquires the calculated feature value, and assigns the time before the frequency transform to the feature value to arrange it in time series. In detail, the time-series arrangement means 108 assigns, to the feature value, the time corresponding to the time-series data acquired by the second sensor 103 and the third sensor 104 to arrange it in time-series. The time-series arrangement means 108 outputs the feature value arranged in time series to the correlation calculation means 109.

The correlation check means 113 checks the correlation between the feature value arranged in time series and the time-series data acquired from the second sensor 103 and the third sensor 104.

The correlation calculation means 109 calculates the correlation between the feature value arranged in time series and the time-series data acquired from the second sensor 103 and the third sensor 104, and stores the calculation result in the correlation storage means 110. The correlation calculation means 109 may perform, other than the correlation calculation, calculation of an invariant expression between a plurality of sets of time-series data according to autoregressive with exogenous (ARX). In the case of calculating the correlation between the plurality of sets of time-series data, the correlation calculation means 109 may perform a process that facilitates the generation of the correlation beforehand, as preprocessing. For example, the correlation calculation means 109 may perform simple averaging, moving averaging, integration, differentiation, etc., as preprocessing. The preprocessing may be carried out by the correlation calculation means 109 or by other means.

Moreover, the correlation calculation means 109 may perform a sorting process of excluding any unpromising correlation from the calculated correlation. For example, in the case of obtaining a plurality of natural frequencies from a sensor and calculating the correlation with another sensor value, the correlation calculation means 109 may keep only the natural frequency with the highest correlation and abandon the correlation calculation results relating to the other natural frequencies. Such a sorting process and process of abandoning part of calculation results may be carried out by the correlation calculation means 109 or by other means.

When storing the calculation result in the correlation storage means 110, the correlation calculation means 109 may transfer the data via a storage medium such as a memory card, or via a network.

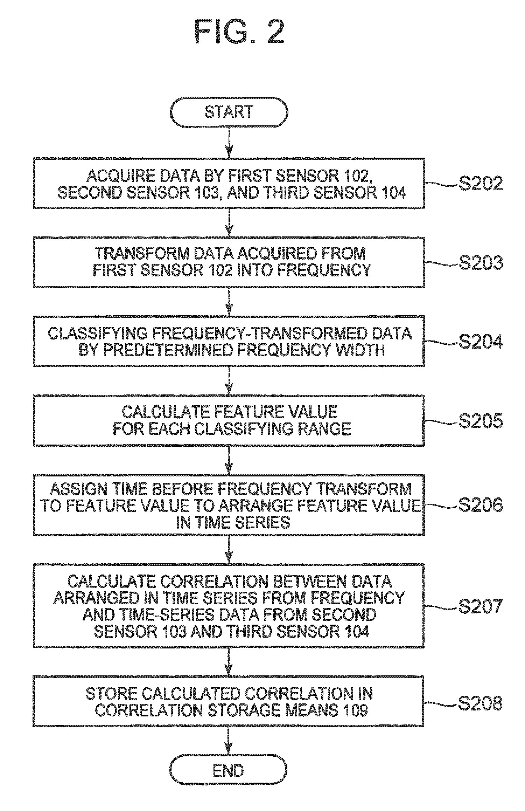

Fig. 2 is a flowchart depicting the operation of the analysis device in the first exemplary embodiment. When the operation starts, first the first sensor 102, the second sensor 103, and the third sensor 104 acquire data (step S202).

The frequency transform means 105 transforms the waveform data acquired from the first sensor 102 into frequency (step S203).

The classifying means 106 classifies the frequency-transformed data by the predetermined frequency width (step S204).

The feature value calculation means 107 calculates the feature value of the data for each classifying range (step S205).

The time-series arrangement means 108 assigns the time before the frequency transform to the calculated feature value, to arrange it in time series (step S206).

The correlation calculation means 109 calculates the correlation between the data of the frequency-based feature value arranged in time series and the time-series data acquired by the second sensor 103 and the third sensor 104 (step S207). The correlation calculation means 109 stores the correlation in the correlation storage means 110 (step S208).

The analysis device 101 in this exemplary embodiment can obtain the correlation between the feature value based on the waveform data acquired from the first sensor 102 and the time-series data acquired from the second sensor 103 and the third sensor 104. In the case where the object is soil, the correlation is disrupted if the object changes from an elastic body to a plastic body. Hence, by continuously acquiring the correlation, the user can detect a change in the state of the object easily and accurately. For example, the analysis device 101 in this exemplary embodiment can detect a change in the state of soil, and so is capable of prediction or early detection of sediment disasters.

The analysis device 101 in this exemplary embodiment is equally capable of prediction or early detection of bridge breakages, road breakages, or tunnel collapses, by detecting concrete or metal degradation based on a correlation change. Iron or concrete expands and contracts depending on temperature, humidity (or rainfall), inclination (in the case of bridges), or pressure (or distortion), and thus changes in natural frequency. Hence, in the case where the natural frequency which changes depending on temperature, humidity, inclination, pressure, etc. deviates from a predicted value by a model created during normal time, it can be estimated that some kind of breakage has occurred or is about to occur.

### Example 1

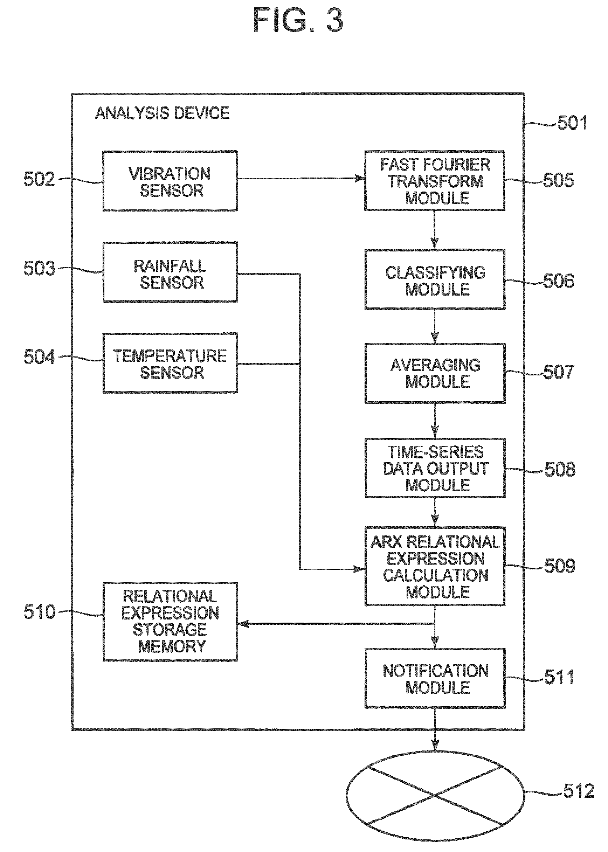

The following describes an example of the analysis device in the first exemplary embodiment. Fig. 3 is a block diagram depicting the structure of the analysis device in Example 1.

An analysis device 501 includes a vibration sensor 502, a rainfall sensor 503, and a temperature sensor 504. The analysis device 501 also includes a fast Fourier transform module 505, a classifying module 506, an averaging module 507, a time-series data output module 508, an ARX relational expression calculation module 509, a relational expression storage memory 510, and a notification module 511. The analysis device 501 is connected to a network 512.

The vibration sensor 502 is connected to the fast Fourier transform module 505. The vibration sensor 502 acquires waveform data of vibration of an object. The waveform data is time-series data.

The rainfall sensor 503 and the temperature sensor 504 are connected to the ARX relational expression calculation module 509. The rainfall sensor 503 acquires time-series data of rainfall. The temperature sensor 504 acquires time-series data of temperature.

The fast Fourier transform module 505 transforms the waveform data of the vibration acquired from the vibration sensor 502 into frequency, and outputs the frequency to the classifying module 506.

The classifying module 506 classifies the frequency-transformed data by a predetermined frequency width, and outputs the data to the averaging module 507. For example, the classifying module 506 decomposes the frequency-transformed data into 9 frequency bands of: less than 10 Hz; 10 Hz or more and less than 20 Hz; 20 Hz or more and less than 30 Hz; 30 Hz or more and less than 40 Hz; 40 Hz or more and less than 50 Hz; 50 Hz or more and less than 100 Hz; 100 Hz or more and less than 200 Hz; 200 Hz or more and less than 300 Hz; and 300 Hz or more. Such decomposition method is defined by the user and stored in the analysis device 501 beforehand (not depicted).

The averaging module 507 calculates the average value of frequency for each classifying range, and outputs the average value to the time-series data output module 508 as a feature value.

The time-series data output module 508 assigns the time before the frequency transform to the average value, to arrange it in time series. The time-series data output module 508 outputs the data arranged in time series to the ARX relational expression calculation module 509.

The ARX relational expression calculation module 509 calculates an ARX relational expression between the vibration data arranged in time series from frequency and the time-series data acquired from the rainfall sensor 503 and the temperature sensor 504. The ARX relational expression calculation module 509 stores the coefficients of the relational expression in the relational expression storage memory 510.

The notification module 511 notifies the waveform data acquired by the vibration sensor 502, the time-series data acquired by any of the rainfall sensor 503 and the temperature sensor 504, the frequency data, the feature value, or the correlation (ARX relational expression or its coefficients) between the feature value and the time-series data, to another information terminal or the like via the network 512.

The network 512 is a communication network for data communication, and is, for example, an Internet connection or a LAN connection.

The analysis device 501 may perform a process of calculating any number of natural frequencies transformed from the vibration sensor 501 and setting such natural frequency as a feature value, instead of the processes by the classifying module 506 and averaging module 507. In this case, the time-series data output module 508 arranges not the averaged data but the natural frequency in time series, and outputs it. The ARX relational expression calculation module 509 keeps only a relational expression highly correlated with rainfall or temperature from among any number of natural frequencies, and stores it in the relational expression storage memory 510. The ARX relational expression calculation module 509 may use, for example, a scale called a fitness value as such correlation. The fitness value denotes the degree of coincidence between a measured value and a predicted value based on a relational expression in a modeling period.

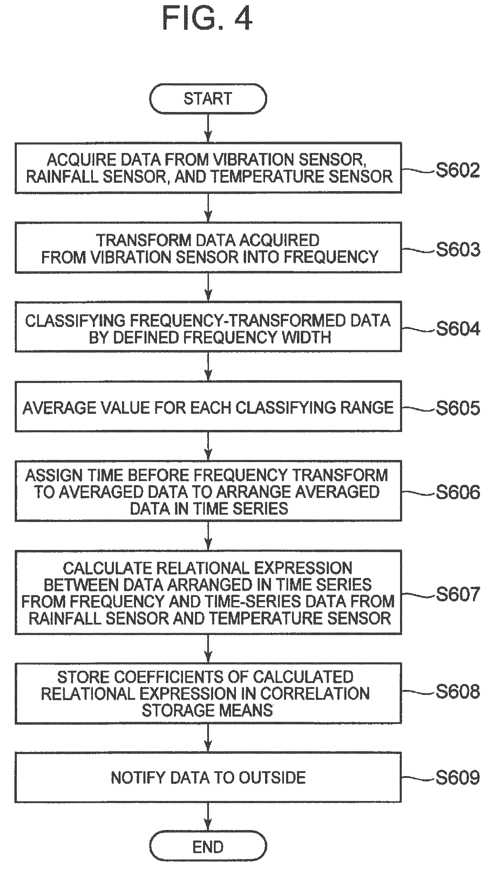

Fig. 4 is a flowchart depicting the operation of the analysis device in Example 1.

When the operation starts, the vibration sensor 502, the rainfall sensor 503, and the temperature sensor 504 acquire time-series data (step S602).

The fast Fourier transform module 505 transforms, from among the time-series data, the waveform data of the vibration acquired from the vibration sensor 502 into frequency (step S603).

The classifying module 506 classifies the frequency-transformed data by the predetermined frequency width (step S604).

The averaging module 507 calculates the average value for each classifying range (step S605).

The time-series data output module 508 assigns the time before the frequency transform to the average value, to transform it back into time-series data (step S606).

The ARX relational expression calculation module 509 calculates an ARX relational expression between the vibration data arranged in time series from frequency and the time-series data acquired from the rainfall sensor 503 and the temperature sensor 504 (step S607). The ARX relational expression calculation module 509 stores the coefficients of the relational expression in the relational expression storage memory 510 as the correlation (step S608).

The notification module 511 notifies the waveform data acquired by the vibration sensor 502, the time-series data acquired by any of the rainfall sensor 503 and the temperature sensor 504, the frequency data, the feature value, or the correlation (ARX relational expression or its coefficients) between the feature value and the time-series data, to another information terminal or the like via the network 512 (step S609).

### Exemplary Embodiment 2

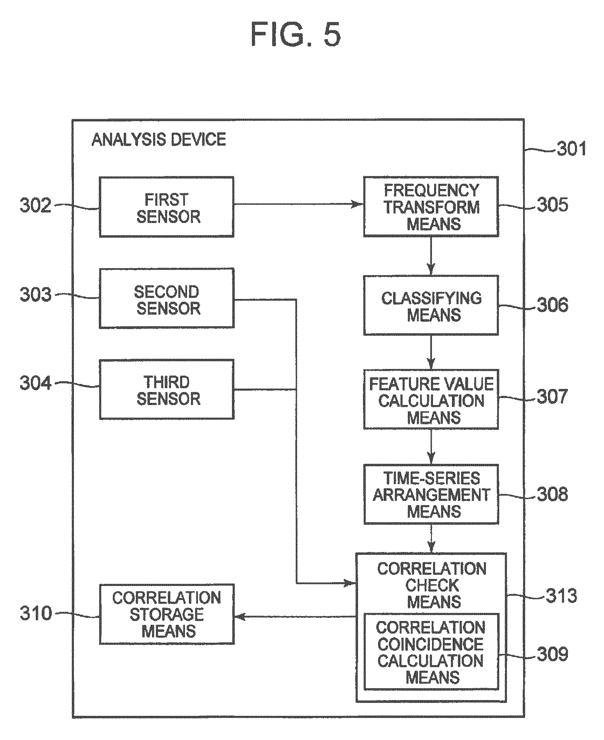

The following describes the structure of an analysis device according to a second exemplary embodiment. Fig. 5 is a block diagram depicting the structure of the analysis device in the second exemplary embodiment. An analysis device 301 in this exemplary embodiment is an analysis device in an observation stage.

The analysis device 301 includes a first sensor 302, a second sensor 303, and a third sensor 304. The analysis device 301 also includes frequency transform means 305, classifying means 306, feature value calculation means 307, time-series arrangement means 308, correlation check means 313, and correlation storage means 310. The correlation check means 313 includes correlation coincidence calculation means 309. The analysis device 301 in this exemplary embodiment has the structure in which the correlation coincidence calculation means 309 is included instead of the correlation calculation means 109 in the analysis device 101 in the first exemplary embodiment.

The frequency transform means 305, the classifying means 306, the feature value calculation means 307, the time-series arrangement means 308, and the correlation check means 313 are realized, for example, by an information processing device such as a central processing unit (CPU) operating according to a program or hardware designed to perform specific computation and the like. The program is computer-readable, and is stored in a non-transitory information storage medium.

The first sensor 302 is connected to the frequency transform means 305. The first sensor 302 acquires waveform data of light, sound, vibration, or the like generated from an object.

The second sensor 303 and the third sensor 304 are connected to the correlation check means 313. The second sensor 303 and the third sensor 304 each acquire time-series data of an element that affects a change in the state of the object. For example, the second sensor 303 and the third sensor 304 acquire time-series data of rainfall, temperature, humidity, sunlight intensity, object inclination, soil moisture content, etc.

The frequency transform means 305 transforms, from among the time-series data, the waveform data acquired from the first sensor 302 into frequency.

The classifying means 306 classifies the frequency-transformed data by a predetermined frequency width, and outputs the data to the feature value calculation means 307. The frequency width may be any frequency width. The analysis device 301 does not necessarily need to include the classifying means 306. For example, in the case where the feature value calculation means 307 calculates a natural frequency as a feature value, the analysis device 301 does not need to perform classifying. The classifying means 306 may exclude unnecessary frequency bands from the classifying process. In this case, subsequent processes such as feature value calculation may be omitted.

The feature value calculation means 307 calculates, for each classifying range, a feature value based on the classified frequency, and outputs the calculated feature value to the time-series arrangement means 308. The feature value calculation means 307 calculates the feature value of each set of data, by calculating the average value, maximum value, dispersion, deviation, root mean square (RMS), etc. of each classifying range. The feature value calculation means 307 may determine a natural frequency (also referred to as a dominant frequency) in the frequency data, and set the frequency value of the natural frequency as the frequency feature value.

The time-series arrangement means 308 assigns the time before the frequency transform to the calculated feature value to arrange it in time series. In detail, the time-series arrangement means 308 assigns, to the feature value, the time corresponding to the time-series data acquired by the second sensor 303 and the third sensor 304 to arrange the feature value in time-series. The time-series arrangement means 308 outputs the time-series data to the correlation coincidence calculation means 309.

The correlation coincidence calculation means 309 calculates the correlation coincidence indicating the degree of coincidence between the correlation between the pre-stored feature value and the time-series data and the correlation between the newly calculated feature value and the time-series data. In detail, based on the waveform data-based feature value and the correlation stored in the correlation storage means 310 beforehand, the correlation coincidence calculation means 309 calculates the predicted value of the time-series data acquired by the second sensor 303 and the third sensor 304, and calculates the degree of coincidence with the measured value. Alternatively, based on the time-series data acquired by the second sensor 303 and the third sensor 304 and the correlation stored in the correlation storage means 310 beforehand, the correlation coincidence calculation means 309 calculates the predicted value of the waveform data-based feature value, and calculates the degree of coincidence with the measured value. The correlation coincidence calculation means 309 may perform, other than the correlation calculation, calculation of an invariant expression between a plurality of sets of time-series data according to ARX.

In the case of calculating the difference between the measured value and the predicated value according to the correlation prepared beforehand, the correlation coincidence calculation means 309 may perform preprocessing. For example, the correlation coincidence calculation means 309 may perform simple averaging, moving averaging, integration, differentiation, etc., as preprocessing. After calculating the correlation coincidence, the correlation coincidence calculation means 309 may perform a process of notifying the correlation coincidence. In the case where the coincidence exceeds or is less than a predetermined threshold, the correlation coincidence calculation means 309 may notify it to the outside. Such preprocessing and notification process may be carried out by the correlation coincidence calculation means 309 or by other means.

The analysis device 301 may have a structure in which the correlation coincidence calculation means 309 is added to the correlation check means 113 in the first exemplary embodiment.

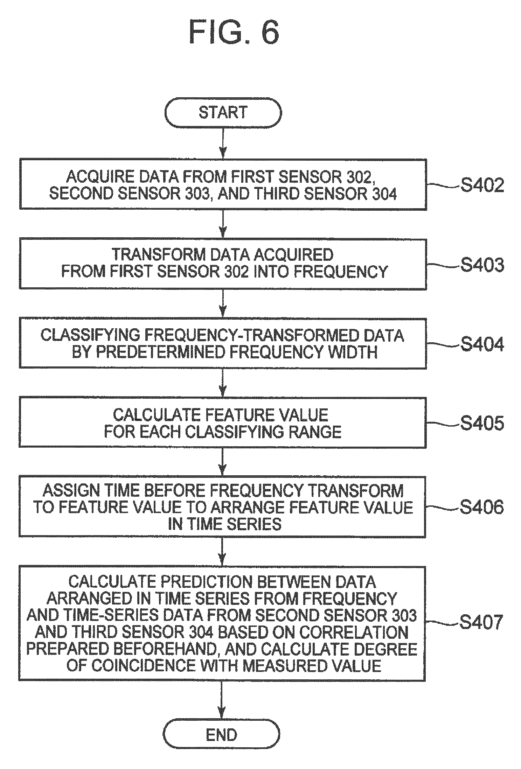

The following describes the operation of the analysis device in the second exemplary embodiment. Fig. 6 is a flowchart depicting the operation of the analysis device in the second exemplary embodiment.

When the operation of the analysis device 301 starts, first the first sensor 302, the second sensor 303, and the third sensor 304 acquire time-series data (step S402).

The frequency transform means 305 transforms, from among the time-series data, the waveform data acquired from the first sensor 302 into frequency (step S403).

The classifying means 306 classifies the frequency-transformed data by the predetermined frequency width (step S404).

The feature value calculation means 307 calculates the feature value for each classifying range (step S405).

The time-series arrangement means 308 assigns the time before the frequency transform to the feature value, to arrange it in time series (step S406).

The correlation coincidence calculation means 309 calculates, for the data arranged in time series from frequency and the time-series data from the second sensor 303 and the third sensor 304, the predicted value based on the correlation prepared in the correlation storage means 310 beforehand, and calculates the degree of coincidence with the measured value (step S407).

The analysis device 301 in this exemplary embodiment can determine whether or not the correlation between the feature value based on the waveform data acquired from the first sensor 302 and the time-series data acquired from the second sensor 303 and the third sensor 304 coincides with the correlation stored previously. The correlation is disrupted if the object changes from an elastic body to a plastic body. Hence, in the case where the correlations do not coincide with each other, the user can recognize that the state of the object has changed to a plastic body. For example, the analysis device 301 can detect a change in the state of soil, and so is capable of prediction or early detection of sediment disasters. The analysis device 301 is equally capable of prediction or early detection of bridge breakages or tunnel collapses, by detecting concrete or metal degradation.

### Example 2

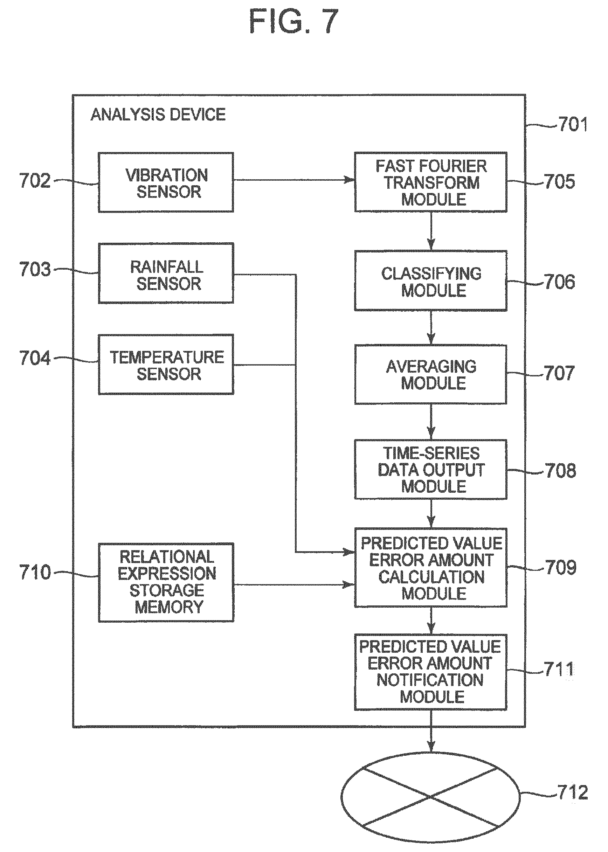

Fig. 7 is a block diagram depicting the structure of an analysis device 701 in Example 2.

The analysis device 701 includes three sensors, i.e. a vibration sensor 702, a rainfall sensor 703, and a temperature sensor 704. The analysis device 701 also includes a fast Fourier transform module 705, a classifying module 706, an averaging module 707, a time-series data output module 708, a predicted value error amount calculation module 709, a relational expression storage memory 710, and a prediction value error amount notification module 711. The prediction value error amount notification module 711 is connected to a network 712.

The vibration sensor 702 is connected to the fast Fourier transform module 705. The vibration sensor 702 acquires waveform data of vibration of an object. The waveform data is time-series data.

The rainfall sensor 703 and the temperature sensor 704 are connected to the predicted value error amount calculation module 709. The rainfall sensor 703 acquires time-series data of rainfall. The temperature sensor 704 acquires time-series data of temperature.

The fast Fourier transform module 705 transforms the data acquired from the vibration sensor 702 into frequency, and outputs the transformed frequency to the classifying module 706.

The classifying module 706 classifies the frequency-transformed data by a predetermined frequency width, and outputs the data to the averaging module 707.

The averaging module 707 calculates the average value for each classifying range, and outputs the average value to the time-series data output module 708.

The time-series data output module 708 assigns the time before the frequency transform to the average value to arrange it in time series, and outputs the data to the predicted value error amount calculation module 709.

The predicted value error amount calculation module 709 calculates an ARX relational expression between the vibration data arranged in time series from frequency and the time-series data acquired from the rainfall sensor 703 and the temperature sensor 704, based on the coefficients of the ARX relational expression stored in the relational expression storage memory 710. The predicted value error amount calculation module 709 thus calculates the error amount between the predicted value and the measured value. The predicted value error amount calculation module 709 outputs the calculation result to the predicted value error amount notification module 711.

The predicted value error amount notification module 711 notifies the acquired error amount to an external information processing terminal or the like (not depicted) via the network 712.

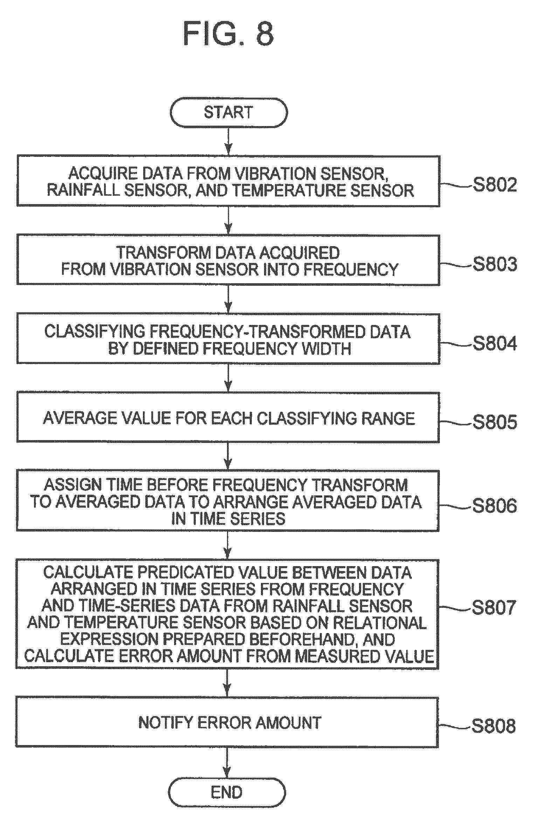

Fig. 8 is a flowchart depicting the operation of the analysis device in Example 2.

When the operation starts, first the vibration sensor 702, the rainfall sensor 703, and the temperature sensor 704 acquire data as time-series data (step S802).

The fast Fourier transform module 705 transforms, from among the time-series data, the waveform data of the vibration acquired from the vibration sensor 702 into frequency (step S803).

The classifying module 706 classifies the frequency-transformed data by the predetermined frequency width (step S804).

The averaging module 707 calculates the average value for each classifying range (step S805).

The time-series data output module 708 assigns the time before the frequency transform to the average value, to arrange it in time series (step S806).

The predicted value error amount calculation module 709 calculates an ARX relational expression between the vibration data arranged in time series from frequency and the time-series data acquired by the rainfall sensor 703 and the temperature sensor 704, based on the coefficients stored in the relational expression storage memory 710. The predicted value error amount calculation module 709 thus calculates the error amount between the predicted value and the measured value (step S807).

The predicted value error amount notification module 711 notifies the error amount to an external information processing terminal or the like (not depicted) via the network 712 (step S808).

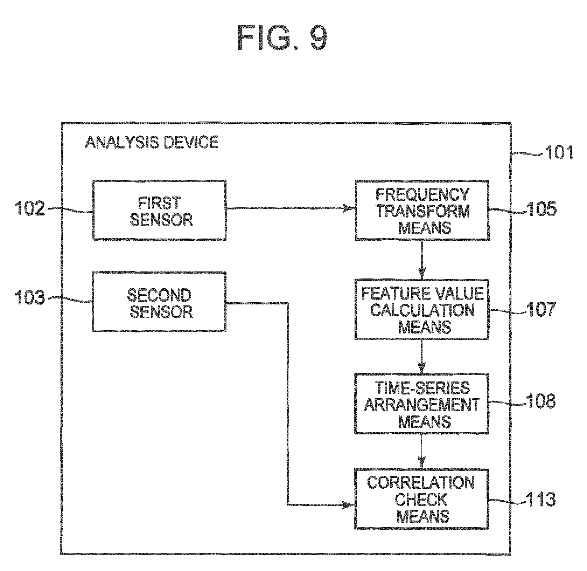

Fig. 9 is a block diagram depicting the structure of main parts of the analysis device according to the present invention. An analysis device 101 according to the present invention includes, as main components: a first sensor 102 for acquiring waveform data generated from an object; a second sensor 103 for acquiring time-series data of an element that affects a change in state of the object; frequency transform means 105 for acquiring a frequency of the waveform data; feature value calculation means 107 for calculating a feature value based on the frequency; time-series arrangement means 108 for assigning a time corresponding to the time-series data, to the feature value; and correlation check means 113 for checking a correlation between the feature value and the time-series data.

An analysis device described in each of the following (1) to (5) is also disclosed in the foregoing embodiments.
(1) An analysis device (e.g. the analysis device 101, the analysis device 301, the analysis device 501, or the analysis device 701) including: a first sensor (e.g. the first sensor 102, the first sensor 302, the vibration sensor 502, or the vibration sensor 702) for acquiring waveform data generated from an object; a second sensor (e.g. the second sensor 103, the third sensor 104, the second sensor 303, the third sensor 304, the rainfall sensor 503, the temperature sensor 504, the rainfall sensor 703, or the temperature sensor 704) for acquiring time-series data of an element that affects a change in state of the object; frequency transform means (e.g. the frequency transform means 105, the frequency transform means 305, the fast Fourier transform module 505, or the fast Fourier transform module 705) for acquiring a frequency of the waveform data; feature value calculation means (e.g. the feature value calculation means 107, the feature value calculation means 307, the averaging module 507, or the averaging module 707) for calculating a feature value based on the frequency; time-series arrangement means (e.g. the time-series arrangement means 108, the time-series arrangement means 308, the time-series data output module 508, or the time-series data output module 708) for assigning a time corresponding to the time-series data, to the feature value; and correlation check means (e.g. the correlation check means 113 or the correlation check means 313) for checking a correlation between the feature value and the time-series data.
(2) In the analysis device, the correlation check means may include correlation calculation means (the correlation calculation means 109 or the ARX relational expression calculation module 509) for calculating the correlation between the feature value and the time-series data.
(3) In the analysis device, the correlation check means may include correlation coincidence calculation means (the correlation coincidence calculation means 309 or the predicted value error amount calculation module 709) for calculating a degree of coincidence between a correlation between a pre-stored feature value and the time-series data and a correlation between a newly calculated feature value and the time-series data.
(4) The analysis device may include classifying means (e.g. the classifying means 106, the classifying means 306, the classifying module 506, or the classifying module 706) for classifying the frequency by a predetermined frequency width, wherein the feature value calculation means calculates the feature value based on the classified frequency.
(5) The analysis device may include notification means (e.g. the notification module 511 or the predicted value error amount notification module), connected to a network (e.g. the network 512 or the network 712) for data communication, for notifying data including any of the waveform data, the time-series data, the feature value, and the correlation between the feature value and the time-series data, to outside via the network.
(6) The analysis device may be an analysis device for detecting a sediment disaster sign, a bridge breakage, or a road breakage based on a change of the correlation, wherein the feature value calculation means calculates a natural frequency as the feature value, and the correlation check means checks the correlation between the natural frequency and the time-series data.

Although the present invention has been described with reference to the foregoing exemplary embodiments and examples, the present invention is not limited to the foregoing exemplary embodiments and examples. Various changes understandable by those skilled in the art can be made to the structures and details of the present invention within the scope of the present invention.

### Industrial Applicability

The present invention is applicable to detection of signs of disasters such as sediment disasters, bridge breakages, and tunnel collapses.

### Reference Signs List

- 101, 301: analysis device
- 102, 302: first sensor
- 103, 303: second sensor
- 104, 304: third sensor
- 105, 305: frequency transform means
- 106, 306: classifying means
- 107, 307: feature value calculation means
- 108, 308: time-series arrangement means
- 109: correlation calculation means
- 309: correlation coincidence calculation means
- 110, 310: correlation storage means
- 113,313: correlation check means

## Claims

1. An analysis device comprising:
a first sensor which acquires waveform data generated from an object;
a second sensor which acquires time-series data of an element that affects a change in state of the object;
frequency transform means for acquiring a frequency of the waveform data;
feature value calculation means for calculating a feature value based on the frequency;
time-series arrangement means for assigning a time corresponding to the time-series data, to the feature value; and
correlation check means for checking a correlation between the feature value and the time-series data.

2. The analysis device according to claim 1, wherein the correlation check means includes correlation calculation means for calculating the correlation between the feature value and the time-series data.

3. The analysis device according to claim 1 or 2, wherein the correlation check means includes correlation coincidence calculation means for calculating a degree of coincidence between a correlation between a pre-stored feature value and the time-series data and a correlation between a newly calculated feature value and the time-series data.

4. The analysis device according to any one of claims 1 to 3, comprising
classifying means for classifying the frequency by a predetermined frequency width,
wherein the feature value calculation means calculates the feature value based on the classified frequency.

5. The analysis device according to any one of claims 1 to 4, comprising
notification means, connected to a network for data communication, for notifying data including any of the waveform data, the time-series data, the feature value, and the correlation between the feature value and the time-series data, to outside via the network.

6. The analysis device according to any one of claims 1 to 5 being an analysis device which detects a sediment disaster sign, a bridge breakage, or a road breakage based on a change of the correlation,
wherein the feature value calculation means calculates a natural frequency as the feature value, and
the correlation check means checks the correlation between the natural frequency and the time-series data.

7. An analysis method comprising:
acquiring waveform data generated from an object;
acquiring time-series data of an element that affects a change in state of the object;
acquiring a frequency of the waveform data;
calculating a feature value based on the frequency;
assigning a time corresponding to the time-series data, to the feature value; and
checking a correlation between the feature value and the time-series data.

8. The analysis method according to claim 7, comprising
calculating the correlation between the feature value and the time-series data.

9. The analysis method according to claim 7 or 8 being an analysis method for detecting a sediment disaster sign, a bridge breakage, or a road breakage based on a change of the correlation, and comprising:
calculating a natural frequency as the feature value; and
checking the correlation between the natural frequency and the time-series data.

10. An analysis program for causing a computer to execute:
a frequency transform process of acquiring a frequency of waveform data generated from an object;
a feature value calculation process of calculating a feature value based on the frequency;
a time-series arrangement process of assigning a time corresponding to time-series data of an element that affects a change in state of the object, to the feature value; and
a correlation check process of checking a correlation between the feature value and the time-series data.

11. The analysis program according to claim 10, causing the computer to, in the correlation check process, calculate the correlation between the feature value and the time-series data.

12. The analysis program according to claim 10 or 11 being an analysis program for causing the computer to execute a process of detecting a sediment disaster sign, a bridge breakage, or a road breakage based on a change of the correlation, and causing the computer to:
in the feature value calculation process, calculate a natural frequency as the feature value; and
in the correlation check process, check the correlation between the natural frequency and the time-series data.
